# EUROPEAN PATENT SPECIFICATION

(11) **EP 3 796 926 B1**
(45) Date of publication and mention of the grant of the patent: **11.05.2022**
(21) Application number: 19727955.7
(22) Date of filing: 21.05.2019
(51) Int. Cl.: A61Q 19/08, A61K 8/11, A61K 8/55, A61K 8/64, A61K 8/73, A61K 9/50, A61K 9/51

(54) **PEPTIDES ENCAPSULATED IN LECITHIN AND ALGINATE**
PEPTIDE - VERKAPSELT IN LECITHIN UND ALGINAT
PEPTIDES ENCAPSULÉS PAR UNE COUCHE CONTENANT DE LA LECITHINE ET DE L'ALGINATE

(30) Priority: 22.05.2018 ES 201830491
(43) Date of publication of application: 31.03.2021
(60) Divisional application: 22152681.7
(73) Proprietor: Infinitec Activos S.L., 08170 Montornés del Vallès, Barcelona (ES)
(72) Inventor: MOURELLE MANCINI, Marisabel, 08028 Barcelona (ES); VASCONCELOS PACHECO, Aimee, 08028 Barcelona (ES)
(74) Representative: Evonik Patent Association
(86) International application number: PCT/EP2019/063071
(87) International publication number: WO 2019/224186

(56) References cited:
- EP-A2- 2 263 643
- WO-A1-2009/062254
- CN-A- 105 853 245
- KR-B1- 101 810 160

## Description

### Field of the Invention

The present invention relates to sub-micrometer capsules comprising a pentapeptide, alginate, and lecithin, compositions comprising them, use thereof in cosmetic treatments, as well as methods of obtaining said capsules.

### Background of the Invention

The first noticeable sign of skin aging is the formation of fine lines and wrinkles. These fine lines appear in different facial areas and are the most easily recognized signs of aging. Fine lines are the first to appear. These small, somewhat shallow wrinkles tend to be noticed in the outer corners of the eyes. They are also known as laugh lines or crow's feet. Fine lines can also be located on the cheeks. On the forehead, wrinkles are noticed as horizontal lines and may be brought out by facial expressions, and they tend to become deeper over time. Smaller sized vertical lines between the eyebrows are caused by furrowing the brow.

There are a number of cosmetic products on the market to improve the appearance of the skin with respect to aging, particularly with respect to wrinkles.

The topical administration of cosmetic or therapeutic agents is a challenge in the field of cosmetic or pharmaceutical formulations since the skin has low permeability to high molecular weight molecules.

The main function of the skin is precisely the regulation of the entrance of substances into the body. The skin of mammals consists of two main layers: the epidermis and the dermis. The epidermis is formed by the stratum corneum, stratum granulosum, stratum spinosum, and stratum basale, the stratum corneum constituting the surface of the skin and the stratum basale constituting the deepest part of the epidermis. The stratum corneum is the layer of skin which contributes the most to the barrier properties and which most obstructs the passage of cosmetically active substances to the deeper layers.

Various systems for traversing the stratum corneum and achieving penetration of cosmetic or therapeutic agents in deeper layers of the skin have been studied. One of these systems is the use of nanomaterials such as nanoparticles [Baroli, B. J. Pharm. Sci., 2010, 99(1), 21-50], in the form of both nanospheres and nanocapsules [Rangari A.T. and Ravikumar P., Asian Journal of Biomedical and Pharmaceutical Sciences, 2015, 5(47), 05-12].

The possibility of using certain short peptides as compounds in cosmetic compositions has been known for some time. Examples of said peptides having anti-wrinkle activity are acetyl hexapeptide-3, palmitoyl tetrapeptide-7, trifluoroacetyl-tripeptide-2, palmitoyl tripeptide-1, palmitoyl tripeptide-38, palmitoyl hexapeptide-14, palmitoyl pentapeptide-4, and palmitoyl hexapeptide-26 is a peptide having antimicrobial activity. Palmitoyl pentapeptide-4 (PP-4) is a peptide of formula Palm-L-Lys-L-Thr-L-Thr-L-Lys-L-Ser-OH (Palm-SEQ ID NO: 1). This peptide is also known by the name Matrixyl^{®}. Said peptide and its anti-wrinkle activity have been described in WO 00/15188 A1 which discloses certain oligopeptides and their use to improve skin appearance. Said patent application also discloses that said peptides might be used in various forms including in the form of macro-, micro- and nanocapsules.

The objective of the present invention is to provide improved compositions comprising peptides in the form of sub-micrometer capsules, which are suitable for the treatment of wrinkles. The compositions of the present invention are characterized by having a high penetration and good stability.

### Summary of the Invention

The inventors have discovered that encapsulating pentapeptides such as PP-4 in lecithin and alginate achieves penetration in deep layers of the skin. Said penetration is surprisingly much greater than that of the same peptides, in particular PP-4 peptide, encapsulated in alginate but using a surfactant other than the lecithin, as shown in the examples. Furthermore, the improvement in cutaneous penetration of the peptides, in particular PP-4 peptide encapsulated in lecithin and alginate is greater than the sum of the increase in cutaneous penetration of the peptides, in particular PP-4 peptide, when encapsulated in lecithin alone and when encapsulated in alginate alone, i.e., there is a synergistic effect on the increase in cutaneous penetration. The encapsulation of peptides, in particular PP-4 peptide in lecithin and alginate also provides an unexpected stability to those peptides, in particular to PP-4 peptide, particularly against the enzymatic degradation by proteases. All these advantages obtained when encapsulating the peptides, in particular PP-4 peptide in lecithin and alginate are completely unexpected since the state of the art provides no indication whatsoever which allows determining which specific encapsulation agents are capable of yielding peptide formulations with good penetration and stability.

Therefore in a first aspect, the present invention relates to a sub-micrometer capsule comprising alginate, lecithin, a divalent metal cation, and a peptide selected from the group consisting of a pentapeptide, a hexapeptide, a tetrapeptide, a tripeptide, a dipeptide, and mixtures thereof.

In a second aspect, the present invention relates to a composition comprising the capsule defined in the first aspect and water.

In a third aspect, the present invention relates to the use of the capsule as defined in the first aspect in the preparation of a composition as defined in the second aspect.

In a fourth aspect, the present invention relates to the (cosmetic) use of the capsule as defined in the first aspect or of a composition as defined in the second aspect for skin care.

In a fifth aspect, the present invention relates to a method for obtaining the capsule as defined in the first aspect, which comprises:
(a) preparing an aqueous suspension comprising lecithin and the peptide;
(b) preparing an aqueous solution comprising a source of alginate,
(c) mixing the suspension obtained in step (a) with the solution obtained in step (b), and
(d) adding an aqueous solution comprising a source of divalent metal cation to the mixture obtained in step (c).

In a sixth aspect, the present invention relates to a method for the cosmetic treatment of wrinkles and/or for the cosmetic prevention of the onset of wrinkles in a human subject wherein the capsules of the first aspect of the invention or the compositions of the second aspect of the invention are applied to the skin of said subject.

### Detailed Description of the Invention

### Capsules of the invention

In a first aspect, the present invention relates to a sub-micrometer capsule comprising alginate, lecithin, a divalent metal cation, and a pentapeptide.

In the context of the present invention, the term "peptide" refers to molecules formed by amino acids linked by peptide bonds, preferably said amino acids are selected from the group consisting of lysine (Lys), threonine (Thr), serine (Ser), aspartic acid (Asp), methionine (Met), asparagine (Asn), valine (Val), glycine (Gly), alanine (Ala), proline (Pro), phenylalanine (Phe), and hydroxyproline (Hyp), preferably selected from the group consisting of lysine (Lys), threonine (Thr), and serine (Ser).

Furthermore, the peptides can have the C-terminus and/or N-terminus derivatized, for example, with a C₈-C₂₂ acyl group (such as palmitoyl and octanoyl) at the N-terminus or with an -NH-(CH₂)₃-O-(CH₂)₂-O-(CH₂)₂-O-(CH₂)-NH₂ group at the C-terminus.

The term pentapeptide refers to a peptide as defined above formed by 5 amino acids. The term hexapeptide refers to a peptide as defined above formed by 6 amino acids. The term tetrapeptide refers to a peptide as defined above formed by 4 amino acids. The term tripeptide refers to a peptide as defined above formed by 3 amino acids. The term dipeptide refers to a peptide as defined above formed by 2 amino acids.

In a preferred embodiment, the peptide of the capsules of the invention is selected from the group consisting of: R₁-L-Lys-L-Thr-L-Thr-L-Lys-L-Ser-OH (R₁-SEQ ID NO: 1), wherein R₁ is a C₈-C₂₂ acyl moiety, preferably a palmitoyl or octanoyl moiety.

In a more preferred embodiment, the peptide of the capsules of the invention is a pentapeptide, even more preferably it is a peptide of formula (I):

R₁-L-Lys-L-Thr-L-Thr-L-Lys-L-Ser-OH (R₁-SEQ ID NO: 1) (I)

wherein R₁ is a C₈-C₂₂ acyl moiety, preferably a palmitoyl moiety.

In the context of the present description, a hexapeptide is a peptide of formula (II):

R₁-L-Lys-L-Ala-L-Gln-L-Lys-L-Arg-L-Phe-NH-(CH₂)₃-O-(CH₂)₂-O- (CH₂)₂-O-(CH₂)-NH₂ (R₁-SEQ ID NO: 2-NH-(CH₂)₃-O-(CH₂)₂-O-(CH₂)₂-O- (CH₂)-NH₂), (II)

wherein R₁ is a C₈-C₂₂ acyl moiety, preferably a palmitoyl moiety.

In the context of the present description, a hexapeptide is more preferably a peptide of formula (III):

R₁-L-Asp-L-Asp-L-Met-L-Asn-L-Lys-L-Lys-NH-(CH₂)₃-O-(CH₂)₂-O- (CH₂)₂-O-(CH₂)-NH₂ (R₁-SEQ ID NO: 3-NH-(CH₂)₃-O-(CH₂)₂-O-(CH₂)₂-O- (CH₂)-NH₂), (III)

wherein R₁ is a C₈-C₂₂ acyl moiety, preferably a palmitoyl moiety.

Furthermore, in the context of the present description, a hexapeptide is more preferably a peptide of formula (IV):

R₁-L-Val-L-Gly-L-Val-L-Ala-L-Pro-L-Gly-NH-(CH₂)₃-O-(CH₂)₂-O- (CH₂)₂-O-(CH₃)-NH₂ (R₁-SEQ ID NO: 4-NH-(CH₂)₃-O-(CH₂)₂-O-(CH₂)₂-O- (CH₂)-NH₂), (IV)

wherein R₁ is a C₈-C₂₂ acyl moiety, preferably an octanoyl moiety.

In the context of the present description, a tripeptide is a peptide of formula (V):

R₁-L-Lys-L-Phe-L-Lys-NH-(CH₂)₃-O-(CH₂)₂-O-(CH₂)₂-O-(CH₂)-NH₂ (V)

wherein R₁ is a C₈-C₂₂ acyl moiety, preferably a palmitoyl moiety.In the context of the present invention, the term "acyl" refers to a Rₐ-C(=O)- group, wherein Rₐ is a C₇-C₂₁ linear alkyl or C₇-C₂₁ linear alkenyl radical, preferably a C₇-C₂₁ linear alkyl radical, more preferably a C₁₃-C₁₇ or C₆-C₈ linear alkyl, more preferably a C₁₅ or C₇ linear alkyl, even more preferably a C₁₃-C₁₇ linear alkyl, even more preferably a C₁₅ linear alkyl.

In the context of the present description, a dipeptide is more preferably a peptide of formula (VI):

L-Pro-L-Hyp (VI)

The term "alkyl" refers to a linear hydrocarbon chain radical consisting of carbon and hydrogen atoms, containing no unsaturations, having the indicated number of carbon atoms, for example from 7 to 21 carbon atoms, preferably from 13 to 17 carbon atoms, or from 6 to 8 carbon atoms, more preferably 15 carbon atoms or 7 carbon atoms, even more preferably from 13 to 17 carbon atoms, even more preferably 15 carbon atoms, and being bound to the molecule by means of a single bond.

The term "alkenyl" refers to a linear or branched hydrocarbon chain radical consisting of carbon and hydrogen atoms, containing at least one unsaturation, having the indicated number of carbon atoms, for example from 7 to 21 carbon atoms, and being bound to the molecule by means of a single bond.

In the context of the present invention, the term "capsule" refers to particles having a core and at least one coating surrounding said core, preferably a core containing a peptide of formulae (I) to (VI) with a coating in the form of a lipid layer comprising lecithin, and wherein said lipid layer is in turn coated by a layer comprising alginate and the divalent metal cation. In the state of the art, the divalent metal cations, particularly calcium cations, are known to interact with guluronic acid monomers of the alginate, forming cavities, said model being known as the egg-box model.

In the context of the present invention, the term "sub-micrometer(s)" refers to the mean diameter of the capsules being less than 1 µm, more preferably less than 0.5 µm. Furthermore, preferably the capsules have a mean diameter not less than 0.1 µm. Therefore, preferably the mean diameter of the capsules is in the range from 0.1 to 1 µm, preferably from 0.1 to 0.8 µm, more preferably from 0.1 to 0.5 µm, more preferably from 0.15 to 0.4 µm. Preferably, the capsules are substantially spherical.

The size of the capsules can be determined by means of conventional methods in the art, particularly by means of dynamic light scattering (DLS). First the samples are prepared to determine particle size. To that end, a 100 µL aliquot of the sample to which 900 µL of milli-Q H₂O are added is collected with an automatic pipette. Then the particle size measurement is taken using quartz cuvette and Zetasizer Nano ZS series equipment (Malvern Instruments) of the *Instituto de Química Avanzada de Cataluña* (Advanced Chemistry Institute of Catalonia), CSIC. Particle size measurements can be taken in triplicate for the purpose of demonstrating the reproducibility thereof.

Alginates are a class of biopolymers used in a wide range of applications in the food industry, cosmetic industry, and pharmaceutical industry. Alginic acid is an anionic polysaccharide widely distributed on the cell walls of brown marine algae (*Phaeophyceae*)*.* Said algae are extracted with diluted alkaline solution which solubilizes the alginic acid. Free alginic acid is obtained by means of treatment of the resulting thick viscous mass with mineral acids. Alginate can be obtained after converting the alginic acid to a salt, preferably the sodium salt (sodium alginate). In the context of the present invention, the term "alginate" refers to the anion of alginic acid. As one skilled in the art will understand, said alginates can be provided by a suitable source, such as a salt of the alginic acid. The most common salts are sodium salt, potassium salt, and a mixture thereof. In a preferred embodiment, the source of alginate is sodium alginate. Alginate is a linear polymer formed by anions of β-D-mannuronic acid (M, β-D-mannuronate) and of α-L-guluronic acid (G, α-L-guluronate) bound by means of glycosidic bonds (1-4). Preferably, the β-D-mannuronate and α-L-guluronate units are in the form of blocks of G monomers (for example, -GGGGG-) and M monomers (for example, -MMMMM-), i.e., monomers of one and the same type are grouped together, said blocks being separated by a block of G and M monomers arranged in an alternating manner (for example, - GMGMGM-) and/or randomly (for example, -GGMGMMG-). Alginates form gels with divalent metal cations, such as calcium, following the model known as the egg-box. According to said model proposed by Grant in 1973, when two chains of G blocks are lined up side by side, it creates a gap which has the ideal dimension for the coordination of a divalent metal cation, particularly calcium. Preferably, the average molecular weight of the alginate is from 250 to 650 g/mol, preferably from 300 to 500 g/mol, more preferably from 350 to 450 g/mol, more preferably from 380 to 420 g/mol, even more preferably from 395 to 415 g/mol, most preferably from 400 to 410 g/mol.

In the context of the present invention, the term "lecithin" refers to a mixture of substances comprising phospholipids and carbohydrates. Phospholipids are the major component of lecithin. Lecithin has amphiphilic properties and therefore can be used as a surfactant. The lecithin can be obtained from several sources, such as for example soy, eggs, milk, colza, cotton, wheat, corn, and sunflower, among others. Preferably, the lecithin used in the present invention is soy lecithin.

The term "phospholipid" refers to a lipid containing glycerol bound by means of ester bonds to two fatty acid groups and to a phosphate group, said phosphate group optionally being bound through a phosphoester bond to another group such as a choline, ethanolamine, inositol or serine, or to lipids having a phosphocholine or phosphoethanolamine fragment bound through an ester bond to the 1-hydroxyl group of a ceramide (the ceramide being a sphingosine bound to a fatty acid). Examples of phospholipids are phosphatidylcholine (PC), lysophosphatidylcholine (LPC), phosphatidylethanolamine (PE), phosphatidylinositol (PI), phosphatidylserine (PS), and phosphatidic acids, the structures of which are shown below and wherein each R independently represents a fatty acid. Preferably, the lecithin comprises one or more phospholipids selected from the group consisting of phosphatidylcholine, lysophosphatidylcholine, phosphatidylethanolamine, phosphatidylinositol, and phosphatidic acid. More preferably the lecithin comprises phosphatidylcholine, lysophosphatidylcholine, phosphatidylethanolamine, phosphatidylinositol, and phosphatidic acid. Even more preferably, the lecithin comprises 18-25% by weight of phosphatidylcholine, 2-6% by weight of lysophosphatidylcholine, 10-16% by weight of phosphatidylethanolamine, 14-19% by weight of phosphatidylinositol, 2-9% by weight of phosphatidic acid, wherein the percentages by weight are expressed with respect to the total weight of the lecithin.

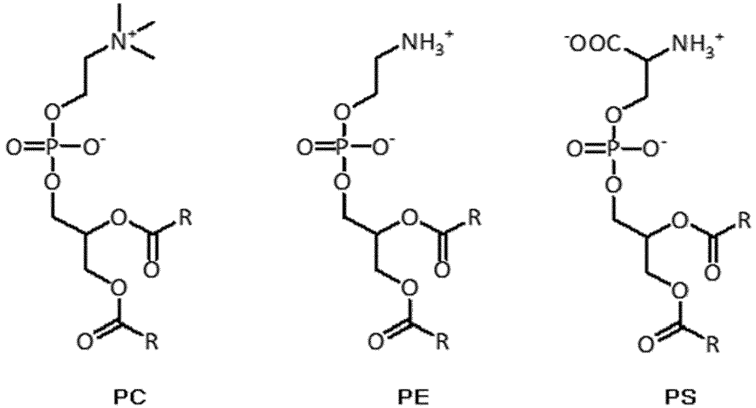

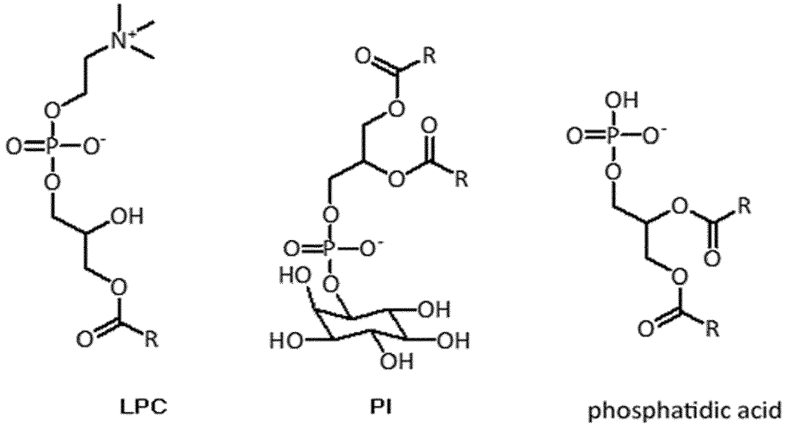

The term "fatty acid" refers to a saturated or unsaturated linear hydrocarbon chain having 6 or more carbon atoms in the chain, such as from 6 to 26 carbon atoms for example, and having a carboxylic acid group at one of the ends of the chain. Examples of saturated fatty acids are caprylic, capric, lauric, myristic, palmitic, stearic, arachidic, behenic, lignoceric, cerotic acids, and the like. Unsaturated fatty acids have one or more double bonds (C=C), preferably one, two, three, four, five, or six double bonds. Examples of unsaturated fatty acids are myristoleic, palmitoleic, sapienic, oleic, elaidic, vaccenic, linoleic, linoelaidic, γ-linolenic, arachidonic, eicosapentaenoic, erucic, docosahexaenoic, hexadecatrienoic, stearidonic, eicosatrienoic, eicosatetraenoic, heneicosapentaenoic, docosapentaenoic, tetracosapentaenoic, tetracosahexaenoic, calendic, eicoadienoic, dihomo-γ-linolenic, docosadienoic, adrenic, docosapentaenoic, tetracosatetraenoic, gondoic, nervonic acids, and the like. Preferably, the lecithin phospholipid fatty acids are selected from the group consisting of stearic acid, palmitic acid, oleic acid, linoleic acid, and mixtures thereof.

In the context of the present invention, the term "carbohydrate" refers to a molecule made up of carbon, oxygen and hydrogen with empirical formula Cₘ(H₂O)ₙ wherein n and m are integers, such as monosaccharides, disaccharides (formed by 2 monosaccharide units), oligosaccharides (formed by between 3 and 10 monosaccharide units), and polysaccharides (formed by more than 10 monosaccharide units). Examples of monosaccharides are trioses, such as glyceraldehyde; tetroses, such as erythrose and threose; pentoses, such as ribose, deoxyribose, arabinose, xylose, and lyxose; hexoses, such as alose, altrose, glucose, gulose, mannose, idose, galactose, and talose; and ketoses, such as dihydroxyacetone, erythrulose, ribulose, xylulose, psicose, fructose, sorbose, and tagatose. The disaccharides are formed by the binding of two identical or different monosaccharides by the formation of a glycosidic bond, i.e., condensation between the hemiacetal group of one of the monosaccharides and a hydroxyl group of the other monosaccharide with the removal of a water molecule. Examples of disaccharides are sucrose, lactose, lactulose, maltose, isomaltose, maltulose, trehalose, celobiose, laminaribiose, gentibiose, turanose, palatinose, gentiobioulose, manobiose, melibiose, melibiulose, rutinose, rutinolose, and xylobiose. The oligosaccharides are formed by the binding of between 3 and 10 identical or different monosaccharides, by the formation of glycosidic bonds between adjacent monosaccharides. Examples of oligosaccharides are inulin, fructooligosaccharides, xylooligosaccharides, maltooligosaccharides, galactooligosaccharides, β-glucan, and glycosaminoglycans. Polysaccharides are formed by more than 10 identical or different monosaccharide units by the formation of glycosidic bonds between adjacent monosaccharides. Examples of polysaccharides are maltodextrin, starch, glycogen, cellulose, and chitin. Preferably, the carbohydrate of the lecithin is maltodextrin.

Preferably, the lecithin comprises 18-25% by weight of phosphatidylcholine, 2-6% by weight of lysophosphatidylcholine, 10-16% by weight of phosphatidylethanolamine, 14-19% by weight of phosphatidylinositol, 2-9% by weight of phosphatidic acid and 18-25% by weight of carbohydrates (preferably of the 18-25% by weight of maltodextrin), wherein the percentages by weight are expressed with respect to the total weight of the lecithin.

As it is used herein, a "divalent metal cation" includes any cation of a metallic element the valence of which is 2, for example, cation of an alkaline earth metal, for example calcium, magnesium, and zinc, or if the metallic element has several valences, one of them is 2, for example iron, on the proviso that it is cosmetically acceptable. In a particular embodiment, said divalent metal cation is selected from the group consisting of Ca²⁺, Mg²⁺, Sr²⁺, Ba²⁺, and combinations thereof. Preferably, the divalent metal cation is calcium cation (Ca²⁺).

As one skilled in the art will understand, the divalent metal cation can be provided by a source of said suitable metal cation, such as a compound which generates said divalent metal cation in aqueous solution, for example, sources of calcium cation are calcium chloride, calcium acetate, calcium gluconate, calcium lactate, calcium sorbate, calcium ascorbate, calcium citrate, calcium propionate, calcium sulfate, etc., or mixtures of said compounds, preferably calcium chloride.

In a particular embodiment, the proportion by weight of peptide with respect to alginate in the capsules of the invention is from 0.0001 to 1.5 grams of peptide per gram of alginate. In a preferred embodiment, the proportion by weight of peptide with respect to alginate in the capsules of the invention is from 0.0001 to 0.01 grams of peptide per gram of alginate; preferably from 0.001 to 0.01 grams of peptide per gram of alginate; more preferably from 0.003 to 0.007 grams of peptide per gram of alginate; even more preferably about 0.005 grams of peptide per gram of alginate.

In a particular embodiment, the proportion by weight of a peptide of formulae (I) to (VI) with respect to alginate in the capsules of the invention is from 0.0001 to 1.5 grams of peptide of formulae (I) to (VI) per gram of alginate. In a preferred embodiment, the proportion by weight of a peptide of formulae (I) to (VI) with respect to alginate in the capsules of the invention is from 0.0001 to 0.01 grams of peptide of formulae (I) to (VI) per gram of alginate; preferably from 0.001 to 0.01 grams of peptide of formulae (I) to (VI) per gram of alginate; more preferably from 0.003 to 0.007 grams of peptide of formulae (I) to (VI) per gram of alginate; even more preferably about 0.005 grams of peptide of formulae (I) to (VI) per gram of alginate.

In a particular embodiment, the proportion by weight of a peptide of formulae (I) to (VI), wherein R₁ is palmitoyl or octanoyl with respect to alginate in the capsules of the invention is from 0.0001 to 1.5 grams of peptide of formulae (I) to (VI), wherein R₁ is palmitoyl or octanoyl per gram of alginate. In a preferred embodiment, the proportion by weight of a peptide of formulae (I) to (VI), wherein R₁ is palmitoyl or octanoyl with respect to alginate in the capsules of the invention is from 0.0001 to 0.01 grams of peptide of formulae (I) to (VI), wherein R₁ is palmitoyl or octanoyl per gram of alginate; preferably from 0.001 to 0.01 grams of peptide of formulae (I) to (VI), wherein R₁ is palmitoyl or octanoyl per gram of alginate; more preferably from 0.003 to 0.007 grams of peptide of formulae (I) to (VI), wherein R₁ is palmitoyl or octanoyl per gram of alginate; even more preferably about 0.005 grams of peptide of formulae (I) to (VI) wherein R₁ is palmitoyl or octanoyl per gram of alginate per gram of alginate.

In a particular embodiment, the proportion by weight of peptide with respect to lecithin is from 0.0001 to 0.6 grams of peptide per gram of lecithin. In another preferred embodiment, the proportion by weight of peptide with respect to lecithin is from 0.0001 to 0.01 grams of peptide per gram of lecithin, preferably from 0.001 to 0.01 grams of peptide per gram of lecithin; more preferably from 0.001 to 0.003 grams of peptide per gram of lecithin; even more preferably about 0.0025 grams of peptide per gram of lecithin.

In a particular embodiment, the proportion by weight of a peptide of formulae (I) to (VI) with respect to lecithin is from 0.0001 to 0.6 grams of peptide of formulae (I) to (VI) per gram of lecithin. In another preferred embodiment, the proportion by weight of a peptide of formulae (I) to (VI) with respect to lecithin is from 0.0001 to 0.01 grams of peptide of formulae (I) to (VI) per gram of lecithin, preferably from 0.001 to 0.01 grams of peptide of formulae (I) to (VI) per gram of lecithin; more preferably from 0.001 to 0.003 grams of peptide of formulae (I) to (VI) per gram of lecithin; even more preferably about 0.0025 grams of peptide of formulae (I) to (VI) per gram of lecithin.

In another particular embodiment, the proportion by weight of a peptide of formulae (I) to (VI), wherein R₁ is palmitoyl or octanoyl with respect to lecithin is from 0.0001 to 0.6 grams of peptide of formulae (I) to (VI), wherein R₁ is palmitoyl or octanoyl per gram of lecithin. In another preferred embodiment, the proportion by weight of a peptide of formulae (I) to (VI), wherein R₁ is palmitoyl or octanoyl with respect to lecithin is from 0.0001 to 0.01 grams of peptide of formulae (I) to (VI), wherein R₁ is palmitoyl or octanoyl per gram of lecithin, preferably from 0.001 to 0.01 grams of peptide of formulae (I) to (VI), wherein R₁ is palmitoyl or octanoyl per gram of lecithin; more preferably from 0.001 to 0.003 grams of peptide of formulae (I) to (VI), wherein R₁ is palmitoyl or octanoyl per gram of lecithin; even more preferably about 0.0025 grams of peptide of formulae (I) to (VI), wherein R₁ is palmitoyl or octanoyl per gram of lecithin.

In another particular embodiment, a proportion by weight of peptide with respect to divalent metal cation is from 0.1 to 150 grams of peptide per gram of divalent metal cation. In another preferred embodiment, a proportion by weight of peptide with respect to divalent metal cation is from 0.1 to 1 gram of peptide per gram of divalent metal cation; preferably from 0.5 to 1 gram of peptide per gram of divalent metal cation; more preferably from 0.6 to 0.8 grams of peptide per gram of divalent metal cation.

In another particular embodiment, a proportion by weight of a peptide of formulae (I) to (VI) with respect to divalent metal cation is from 0.1 to 150 grams of peptide of formulae (I) to (VI) per gram of divalent metal cation. In another preferred embodiment, a proportion by weight of a peptide of formulae (I) to (VI) with respect to divalent metal cation is from 0.1 to 1 gram of peptide of formulae (I) to (VI) per gram of divalent metal cation; preferably from 0.5 to 1 gram of peptide of formulae (I) to (VI) per gram of divalent metal cation; more preferably from 0.6 to 0.8 grams of peptide of formulae (I) to (VI) per gram of divalent metal cation.

In another preferred embodiment, a proportion by weight of a peptide of formulae (I) to (VI), wherein R₁ is palmitoyl or octanoyl with respect to calcium cation is from 0.1 to 150 grams of peptide of formulae (I) to (VI), wherein R₁ is palmitoyl or octanoyl per gram of calcium cation. In another preferred embodiment, a proportion by weight of a peptide of formulae (I) to (VI), wherein R₁ is palmitoyl or octanoyl with respect to calcium cation is from 0.1 to 1 gram of peptide of formulae (I) to (VI), wherein R₁ is palmitoyl or octanoyl per gram of calcium cation; preferably from 0.5 to 1 gram of peptide of formulae (I) to (VI), wherein R₁ is palmitoyl or octanoyl per gram of calcium cation; more preferably from 0.6 to 0.8 grams of peptide of formulae (I) to (VI), wherein R₁ is palmitoyl or octanoyl per gram of calcium cation.

In the context of the present invention, the term "about" must be interpreted to mean the value indicated ± 5% of said value.

In the context of the present invention, the term "cosmetically acceptable" refers to molecular entities and compositions which are physiologically tolerable and do not usually produce an unwanted allergic reaction or the like when administered to a human being.

### Compositions of the invention

In a second aspect, the present invention relates to a composition comprising the capsule defined in the first inventive aspect and water.

In a particular embodiment, said composition is a dispersion of the capsules in water. Due to the sub-micrometer size of the capsules, said dispersion in water forms a colloid. In the context of the present invention, a colloid refers to a system formed by two or more phases, a fluid (liquid) and another dispersed phase in the form of particles, the size of which is as defined above for the capsules of the invention.

In a preferred embodiment, the composition contains at least 90% of water with respect to the sum of the weight of water and capsules in the composition, preferably at least 95%, more preferably at least 99%, most preferably at least 99.5%.

In another preferred embodiment, the composition of the present invention comprises:
- from 0.001% to 0.1% of peptide,
- from 0.01% to 5% of alginate,
- from 0.0001% to 0.1% of divalent metal cation,
- from 1% to 5% of lecithin, and
- water,
wherein the percentage is expressed with respect to the total of the composition.

In another preferred embodiment, the composition of the present invention comprises:
- from 0.001% to 0.1% of peptide of formulae (I) to (VI),
- from 0.01% to 5% of alginate,
- from 0.0001% to 0.1% of divalent metal cation,
- from 1% to 5% of lecithin, and
- water,
wherein the percentage is expressed with respect to the total of the composition.

In another preferred embodiment, the composition of the present invention comprises:
- from 0.001% to 0.1% of peptide of formulae (I) to (VI), wherein R₁ is palmitoyl or octanoyl,
- from 0.01% to 5% of alginate,
- from 0.0001% to 0.1% of cation calcium,
- from 1% to 5% of lecithin, and
- water,
wherein the percentage is expressed with respect to the total of the composition.

In another preferred embodiment, the compositions of the invention may further comprise a cosmetic agent selected from the group consisting of surfactants, moisturizing agents, antioxidants, emollients, preservatives, humectants, viscosity modifiers, and mixtures thereof.

In the context of the present invention, a "surfactant" is a substance which decreases the surface tension of a composition with respect to the same composition in the absence of said component and furthermore facilitates the uniform distribution of the composition when it is used and it is not lecithin, which is already present in the capsules of the invention. Examples of surfactants suitable for the compositions of the invention are lauryl isoquinolinium bromide and isopropyl alcohol, polysorbate 20, steareth-2 (polyethylene glycol ether (2 units) and stearyl alcohol), oleth-2 (polyethylene glycol ether (2 units) and oleyl alcohol), PEG-8 caprylic/capric glycerides (ethoxylated with 8 units of polyethylene glycol, sodium cocoamphoacetate, coconut oil esters - polyglycerol 6, almond oil esters- PEG-8, ammonium cocosulfate and avocado oil esters - PEG-11, and mixtures thereof.

In the context of the present invention, a "moisturizing agent" refers to a substance which increases the water content of the skin or hair and helps to keep it soft. Examples of moisturizing agents suitable for the compositions of the invention are *vitis vinifera* seed oil, ceramide, glucosylceramide, grape oil esters - PEG-8, glyceryl esters - cocoa butter, shea butter cetyl esters, shea butter glyceride, lauryl cocoate, and mixtures thereof.

In the context of the present invention, an "antioxidant" refers to a substance which inhibits or reduces reactions promoted by oxygen, thereby preventing oxidation and rancidity. Examples of antioxidants suitable for the compositions of the invention are tocopherol, sodium tocopheryl phosphate, 3-glyceryl ascorbate, acetylcysteine, *aloe vera* plant extract, ascorbic acid, ascorbyl dipalmitate, ascorbic acid polypeptide, acetyl trihexylcitrate, ascorbyl linoleate, 2-acetylhydroquinone, apo-lactoferrin, ascorbyl glucoside, ascorbyl lactoside, and mixtures thereof.

In the context of the present invention, an "emollient" refers to a substance which softens the skin. Examples of emollients suitable for the compositions of the invention are apo-lactoferrin, *acacia dealbata* flower wax, acetylarginine, acetylproline, acetylhydroxyproline, acetylated glycol stearate, algae extract, almond oil esters and propylene glycol, aminopropyltocopheryl phosphate, 1,2,6-hexanetriol, and mixtures thereof.

In the context of the present invention, a "preservative" refers to a substance which inhibits the development of microorganisms in the composition. Examples of preservatives suitable for the compositions of the invention are phenoxyethanol; a mixture of caprylyl glycol, glyceryl caprylate, glycerin, and phenylpropanol; a mixture of benzyl alcohol, glyceryl caprylate, and glyceryl undecylenate; a mixture of 2,2-hexanediol and caprylyl glycol; a mixture of phenethyl alcohol, and ethylhexylglycerin; a mixture of pentylene glycol, caprylyl glycol, and ethylhexylglycerin.

In the context of the present invention, a "humectant" refers to a substance which retains humidity. Examples of humectants suitable for the compositions of the invention are 3-glyceryl ascorbate, acetylcyclodextrin, propanediol, algae extract, 2,3-butanediol, 3-ethylhexylglyceryl ascorbate, 3-laurylglyceryl ascorbate, and capryl 3-glyceryl ascorbate.

In the context of the present invention, a "viscosity modifier" refers to a substance which increases the viscosity of a composition, preferably an aqueous composition. Examples of viscosity modifiers suitable for the compositions of the invention are carbomer, sodium carbomer, dextran sulfate sodium, carboxymethyl chitosan, propanediol, carboxymethyl dextran, steareth-30, steareth-40, steareth-50, sodium poly polynaphthalene sulfonate, croscarmelose, sodium glycereth-polyphosphate, and mixtures thereof.

In another preferred embodiment, the compositions of the invention are in the form of a cream, serum, emulsion, gel, foam, paste, ointment, milk, or solution, preferably in the form of cream, solution, serum or gel.

In a third aspect, the capsule of the present invention is used in the preparation of a composition according to the present invention.

Said compositions can be prepared by means of mixing the capsules of the invention with the rest of the components of the corresponding compositions.

### Cosmetic uses of the capsules and compositions of the invention

The peptides of the capsules of the invention as they are defined in the claims, and particularly palmitoyl pentapeptide-4 (PP-4, peptide of formula (I)) exhibit anti-wrinkle activity.

Therefore, in another aspect the present invention relates to the cosmetic (non-therapeutic) use of the capsules or compositions of the invention in skin care. Said use is cosmetic.

In the context of the present invention, the term "care" refers to the maintenance or improvement of the qualities of the skin, such as wrinkles, elasticity, firmness, hydration, shine, tone, or texture, among others, preferably wrinkles.

In a preferred embodiment, skin care is the cosmetic treatment of wrinkles and/or cosmetic prevention of the onset of wrinkles.

In the context of the present invention, the term "treatment" refers to a non-therapeutic cosmetic treatment, in which the application of the composition of the invention on improves the cosmetic appearance of the skin in terms of wrinkles, by either reducing the depth of the wrinkles, reducing the number of wrinkles, or both.

In the context of the present invention, the term "prevention" refers to the capability of the composition of the invention to prevent, delay, or impede the onset of wrinkles in the skin.

### Method of obtaining the capsules of the invention

In another aspect, the present invention relates to a method for obtaining the capsules of the invention, which comprises:
(a) preparing an aqueous suspension comprising lecithin and the peptide;
(b)preparing an aqueous solution comprising a source of alginate,
(c) mixing the suspension obtained in step (a) with the solution obtained in step (b), and
(d) adding an aqueous solution comprising a source of divalent metal cation to the mixture obtained in step (c).

Step (a) of the method is the preparation of an aqueous suspension comprising the lecithin and the peptide, particularly a peptide of formulae (I) to (VI). Preferably, in said peptide of formulae (I) to (VI) wherein the R₁ group is palmitoyl or octanoyl. As explained with respect to the capsules of the invention, the lecithin can be obtained from various sources, such as for example soy, eggs, milk, colza, cotton, and sunflower, among others. Preferably the lecithin used is soy lecithin. The suspension of step (a) can be prepared by simple mixture of the peptide, lecithin, and water, preferably at room temperature (20-25°C), by means of shear homogenization at from 10000 to 30000 rpm, preferably from 20000 to 30000 rpm, more preferably at about 24000 rpm. Preferably between 5 and 15 liters of water are used per gram of peptide, more preferably between 8 and 12 liters of water per gram of peptide.

Step (b) of the method is the preparation of an aqueous solution comprising a source of alginate. As explained with respect to the capsules of the invention, suitable sources of alginate are the salts of alginic acid, such as for example sodium salt, potassium salt, or mixtures thereof. In a preferred embodiment, the source of alginate is sodium alginate. The solution of step (b) can be prepared by simple mixture of the source of alginate and water, preferably at room temperature (20-25°C) and under stirring. Preferably between 10 and 100 milliliters of water are used per gram of alginate, more preferably between 30 and 60 milliliters of water per gram of alginate.

Steps (a) and (b) can be performed in any order, i.e., step (a) can be performed first and then step (b), step (b) can be performed first and then step (a), or steps (a) and (b) can be performed simultaneously.

Step (c) of the method is the mixture of the suspension obtained in step (a) with the solution obtained in step (b). Said step can be performed by addition of the suspension obtained in step (a) to the solution obtained in step (b), by addition of the solution obtained in step (b) to the suspension obtained in step (a), or by simultaneous addition of the suspension obtained in step (a) and the solution obtained in step (b) (in a reaction vessel other than the one for the formation of the suspension of step (a) and for the formation of the solution of step (b)). Preferably, the mixture of step (c) is performed at room temperature (20-25°C) and under stirring.

The final step of the method, step (d), is the addition of an aqueous solution comprising a source of divalent metal cation to the mixture obtained in step (c) for the formation of the capsules of the invention. As explained with respect to the capsules of the invention, a source of divalent metal cation is a compound which generates said divalent metal cation in aqueous solution. Examples of sources of divalent metal cation are calcium chloride, calcium acetate, calcium gluconate, calcium lactate, calcium sorbate, calcium ascorbate, calcium citrate, calcium propionate, calcium sulfate, etc., or mixtures of said compounds, preferably calcium chloride. Preferably, step (d) is performed by means of addition of the aqueous solution comprising the source of divalent metal cation to the mixture of step (c), preferably said addition is performed drop-wise and under stirring. The aqueous solution comprising the source of divalent metal cation can be prepared by mixture of the source of divalent metal cation and water, preferably at room temperature (20-25°C) and under stirring. Preferably between 0.5 and 1.5 liters of water are used per gram of divalent metal cation, more preferably between 0.8 and 1.2 liters of water per gram of divalent metal cation.

Preferably, after the addition of the aqueous solution comprising the source of divalent metal cation, the resulting mixture is kept under stirring, for example at least 30 min.

The following non-limiting examples will additionally illustrate specific embodiments of the invention.

### Examples

Capsules which do not comprise a pentapeptide are not covered by the scope of the claims.

### Examples 1a-1c. Alginate and lecithin capsules loaded with peptide of formula (I) or peptide of formula (II) wherein R₁ is palmitoyl

a) 0.005 g of peptide of formula (I) wherein R₁ is palmitoyl (Matrixyl^{®}, PP-4 peptide) were weighed and dissolved in 49.5 g of distilled water, next 2 g of soy lecithin powder (Epikuron 100P IP by Cargill) were added under stirring at room temperature. The preceding solution was homogenized by means of the shear homogenization method at 24000 rpm using ULTRA-TURRAX model IKA^{®} T25. The obtained suspension was dispersed in a solution of sodium alginate (1 g of sodium alginate (Fagron) in 40 g of distilled water). Next, a solution of calcium chloride (0.02 g of calcium chloride (Sigma Aldrich) in 7.5 g of distilled water) was added dropwise and under stirring. Finally, stirring was continued for 30 minutes to harden the capsules.
b) The same method as the one described above in section a) was followed but using 1 g of peptide of formula (I) wherein R₁ is palmitoyl (Matrixyl^{®}, PP-4 peptide).
c) The same method as the one described above in section a) was followed but using 1g of peptide of formula (II) wherein R₁ is palmitoyl (PH-26 peptide ) (not according to the invention).

### Comparative example 2. Alginate and Tween^{®} 20 capsules loaded with peptide of formula (I) wherein R₁ is palmitoyl

0.005 g of peptide of formula (I) wherein R₁ is palmitoyl (Matrixyl^{®}, PP-4 peptide) were weighed and dissolved in 49.5 g of distilled water, next 2 g of Tween ^{®} 20 (Sigma Aldrich) were added under stirring at room temperature. The preceding solution was homogenized by means of the shear homogenization method at 24000 rpm using ULTRA-TURRAX model IKA^{®} T25. Subsequently, the obtained Matrixyl-Tween^{®} 20 solution was dispersed in a solution of sodium alginate (1 g of sodium alginate (Fagron) in 40 g of distilled water). Next, a solution of calcium chloride (0.02 g of calcium chloride (Sigma Aldrich) in 7.5 g of distilled water) was added drop-wise and under stirring. Finally, stirring was continued for 30 minutes to harden the capsules.

### Comparative example 3. Alginate and Brij^{®} O10 capsules loaded with peptide of formula (I) wherein R₁ is palmitoyl

0.005 g of peptide of formula (I) wherein R₁ is palmitoyl (Matrixyl^{®}, PP-4 peptide) were weighed and dissolved in 49.5 g of distilled water, next 2 g of Brij ^{®} O10 (Sigma Aldrich) were added under stirring at room temperature. The preceding solution was homogenized by means of the shear homogenization method at 24000 rpm using the ULTRA-TURRAX model IKA^{®} T25. Subsequently, the Matrixyl-Brij^{®} O10 solution was dispersed in a solution of sodium alginate (1 g of sodium alginate (Fagron) in 40 g of distilled water). Next, a solution of calcium chloride (0.02 g of calcium chloride (Sigma Aldrich) in 7.5 g of distilled water) was added dropwise and under stirring. Finally, stirring was continued for 30 minutes to harden the capsules.

### Comparative example 4. Alginate capsules loaded with peptide of formula (I) wherein R₁ is palmitoyl

0.005 g of peptide of formula (I) wherein R₁ is palmitoyl (Matrixyl^{®}, PP-4 peptide) were weighed and dissolved in 51.5 g of distilled water. The Matrixyl solution was dispersed in a solution of sodium alginate (1 g of sodium alginate (Fagron) in 40 g of distilled water). Next, a solution of calcium chloride (0.02 g of calcium chloride (Sigma Aldrich) in 7.5 g of distilled water) was added dropwise and under stirring. Finally, stirring was continued for 30 minutes to harden the capsules.

### Comparative example 5. Solution of peptide of formula (I) wherein R₁ is palmitoyl and lecithin

0.005 g of peptide of formula (I) wherein R₁ is palmitoyl (Matrixyl^{®}, PP-4 peptide) were weighed and dissolved in 98 g of distilled water, next 2 g of soy lecithin powder (Epikuron 100P IP by Cargill) were added under stirring at room temperature. The preceding solution was homogenized by means of the shear homogenization method at 24000 rpm using the ULTRA-TURRAX model IKA^{®} T25.

### Comparative examples 6a-6c. Solutions of the peptide of formula (I) or peptide of formula (II) wherein R₁ is palmitoyl

a) 0.005 g of peptide of formula (I) wherein R₁ is palmitoyl (Matrixyl^{®}, PP-4 peptide) were weighed and dissolved in 100 g of distilled water.
b) The same method as the one described above in section a) was followed but using 1 g of peptide of formula (I) wherein R₁ is palmitoyl (Matrixyl^{®}, PP-4 peptide).
c) 0.010 g of peptide of formula (II) wherein R₁ is palmitoyl (PH-26 peptide) were weighed and dissolved in 2 mL of PBS (Phosphate-buffered saline) buffer.

### Example 7. Cutaneous penetration

HPLC analysis was carried out in a Waters 996 instrument with a diode array detector equipped with Waters 2695 separations module and Millenium software; an Xbridge BEH130 C18 4.6x100 mm 3.5 µm reversed phase column by Waters was used. UV detection was performed at 220 nm and a mobile phase B gradient of 5 to 100% was performed for 8 minutes at 1.0 ml/min of flow. Under these conditions, the retention time of the peptide of formula (I) wherein R₁ is palmitoyl (Matrixyl^{®}, PP-4) is 6.4 min. Mobile phase A: 0.045% TFA/H₂O, mobile phase B: 0.036% TFA/acetonitrile.

Cutaneous penetration of the formulations of Example 1a of Comparative examples 4, 5 and 6a was determined using static Franz cells. The pig skin samples used in the experiments were cut into circles 2.5 cm in diameter. The pig skin was mounted on the receptor compartment of a Franz diffusion cell system with the stratum corneum facing up towards the donor compartment. As a solution for the receptor, 18 ml of phosphate buffered saline (PBS) pH 7.4 stirred by a magnetic rod at 700 rpm were used. A 25 µl aliquot of each of the assay formulations (Example 1a and Comparative examples 4, 5 and 6a) was applied on the pig skin. The Franz diffusion cells were kept at 32°C, and after 24 h of incubation, the receptor solution was analyzed by means of HPLC (λ=220 nm) to quantify the amount of peptide of formula (I) wherein R₁ is palmitoyl (Matrixyl^{®}, PP-4 peptide). The assays were performed in triplicate. The results are shown in the table below.

| *Formulation* | *Amount of PP-4 in the receptor after 24 h (%)* | *Observed increase in the amount of PP-4 in the receptor after 24 h* | *Theoretical increase in the amount of PP-4 in the receptor after24 h* |
|---|---|---|---|
| Example 1a | 82.7 | 19.2 | 14.3 |
| Comparative example 4 | 25.0 | 5.8 | - |
| Comparative example 5 | 36.7 | 8.5 | - |
| Comparative example 6a | 4.3 | - | - |

The observed increase in the amount of PP-4 peptide in the receptor for Example 1a and Comparative examples 4-5 was determined as the ratio between the amount of PP-4 peptide in the receptor 24 h after the application of the corresponding formulation and the amount of PP-4 peptide in the receptor 24 h after the application of the formulation of Comparative example 6a. The theoretical increase in the amount of PP-4 peptide in the receptor for Example 1a was calculated as the sum of the increases observed in the amount of PP-4 peptide in the receptor of Comparative examples 4 and 5. As can be seen, there is a larger increase than expected in the penetration of PP-4 peptide when it is encapsulated in lecithin and alginate (example 1a).

Cutaneous penetration of the formulations of Example 1a of Comparative examples 2-3 was also determined using static Franz cells. The pig skin samples used in the experiments were cut into circles 3 cm in diameter. The pig skin was mounted on the receptor compartment of a Franz diffusion cell system with the stratum corneum facing up towards the donor compartment. As a solution for the receptor, 18 ml of phosphate buffered saline (PBS) pH 7.4 stirred by a magnetic rod at 700 rpm were used. A 25 µl aliquot of each of the assay formulations (example 1a and comparative examples 2-3) was applied on the pig skin. The Franz diffusion cells were kept at 32°C, and after 20 h of incubation, the receptor solution was analyzed by means of HPLC (λ=220 nm) to quantify the amount of peptide of formula (I) wherein R₁ is palmitoyl (Matrixyl^{®}, PP-4 peptide). The results are shown in the table below.

| *Formulation* | *Amount of PP-4 in the receptor after 20 h (%)* |
|---|---|
| Example 1a | 98 |
| Comparative example 2 | 22 |
| Comparative example 3 | 21 |

As can be seen, the penetration of the PP-4 peptide when it is encapsulated in lecithin and alginate is much higher than the penetration of said peptide encapsulated in Tween^{®} and alginate and higher than the penetration of the peptide encapsulated in Brij^{®} and alginate.

### Example 8a and 8b. Peptide degradation by proteases

a) 5 mg of each of the formulations of Example 1b and Comparative example 6b were incubated at 40°C with 20 mg of papain. Samples were taken at study times 0 h, 0.3 h, 0.6 h, and 5 h after the addition of papain, centrifuged at 1400 rpm for 1 min, and the supernatant was filtered using a 0.45 µm filter and analyzed by HPLC.
b) 20 mg of papain were added to the formulation of Comparative example 6c, and 1.5 mg of papain were added to a solution of 40 mg of the formulation of Example 1c in 1.0 mL of PBS buffer. Both samples were incubated at 40 °C. Aliquots were taken at study times 0 h, 0.17 h, 0.3 h and 24h, after the addition of papain, centrifuged at 1400 rpm for 1 min, and the supernatant was filtered using a 0.45 µm filter and analyzed by HPLC.

HPLC analysis was carried out in a Waters 996 instrument with a diode array detector equipped with Waters 2695 separations module and Millenium software; an Xbridge BEH130 C18 4.6x100 mm 3.5 µm reversed phase column by Waters was used. UV detection was performed at 220 nm and a mobile phase B gradient of 5 to 100% was performed for 8 minutes at 1.0 ml/min of flow. Under these conditions, the retention time of the peptide of formula (I) wherein R is palmitoyl (Matrixyl^{®}, PP-4) is 6.4 min. Mobile phase A: 0.045% TFA/H₂O, mobile phase B: 0.036% TFA/acetonitrile.

The obtained results are shown in tables 1 and 2 below.

As can be seen in table 1, the peptide of formula (I) wherein R₁ is palmitoyl (Matrixyl^{®}, PP-4) is completely degraded only after 5 hours, while the peptide of formula (I) wherein R₁ is palmitoyl (Matrixyl^{®}, PP-4) encapsulated in lecithin and alginate has an improved stability against degradation with papain.

**Table 1. Results for assay of example 8a**

| Time (h) | % PP-4 peptide | |
|---|---|---|
| | Example 1b | Comparative example 6b |
| 0 | 100 | 100 |
| 0.3 | 90 | 69 |
| 0.6 | 84 | 51 |
| 5 | 76 | 0 |

As can be seen in table 2, the peptide of formula (II) wherein R₁ is palmitoyl (PH-26 peptide) is nearly completely degraded in only 20 minutes (0.3 h), whereas the peptide of formula (II) wherein R₁ is palmitoyl (PH-26 peptide) encapsulated in lecithin and alginate shows an improved stability against degradation with papain with 70% of peptide remaining after 24h.

**Table 2. Results for assay of example 8b (not according to the invention).**

| Time (h) | % PH-26 peptide | |
|---|---|---|
| | Example 1c | Comparative example 6c |
| 0 | 100 | 100 |
| 0.17 | 83 | 0.45 |
| 0.3 | 71 | 0.28 |
| 24 | 70 | 0.16 |

## Claims

1. A capsule having a mean diameter less than 1 µm comprising alginate, lecithin, a divalent metal cation, and a pentapeptide.

2. The capsule according to claim 1, wherein the peptide is a peptide of formula (I):
R₁-L-Lys-L-Thr-L-Thr-L-Lys-L-Ser-OH (R₁-SEQ ID NO: 1) (I)
wherein R₁ is a C₈-C₂₂ acyl moiety.

3. The capsule according to claim 2, wherein the R₁ moiety is a palmitoyl or octanoyl moiety.

4. The capsule according to any of the preceding claims, wherein the lecithin is soy lecithin.

5. The capsule according to any of the preceding claims, **characterized in that** the divalent metal cation is a calcium cation.

6. The capsule according to any of the preceding claims, wherein the proportion by weight of peptide with respect to alginate is from 0.0001 to 0.01 grams of peptide per gram of alginate, the proportion by weight of peptide with respect to lecithin is from 0.0001 to 0.01 grams of peptide per gram of lecithin and the proportion by weight of peptide with respect to divalent metal cation is from 0.1 to 1 gram of peptide per gram of divalent metal cation.

7. The capsule according to any of the preceding claims, **characterized in that** its mean diameter is from 0.1 to 0.5 µm.

8. A composition comprising the capsule as defined in any of the preceding claims and water, the amount of water being preferably at least 90% (w/w) of water with respect to the sum of the weight of water and capsules in the composition.

9. The composition according to claim 8, **characterized in that** it further comprises a cosmetic agent selected from the group consisting of surfactants, moisturizing agents, antioxidants, emollients, preservatives, humectants, viscosity modifiers, and mixtures thereof, said composition being preferably in the form of a cream, serum, emulsion, gel, foam, paste, ointment, milk, or solution.

10. Use of the capsule as defined in any of claims 1 to 7 in the preparation of a composition as defined in any of claims 8 to 9.

11. Cosmetic use of the capsule as defined in any of claims 1 to 7 or of a composition as defined in any of claims 8 to 9 for skin care.

12. Use according to claim 11, wherein skin care is the cosmetic treatment of wrinkles and/or cosmetic prevention of the onset of wrinkles.

13. A method for obtaining the capsule as defined in any of claims 1 to 7, which method comprises:
(a)preparing an aqueous suspension comprising lecithin and the peptide;
(b)preparing an aqueous solution comprising a source of alginate,
(c) mixing the suspension obtained in step (a) with the solution obtained in step (b), and
(d)adding an aqueous solution comprising a source of divalent metal cation to the mixture obtained in step (c) .

14. The method according to claim 13, wherein the source of alginate of step (b) is sodium alginate and the source of divalent metal cation is calcium chloride.

15. A method for the cosmetic treatment of wrinkles and/or for the cosmetic prevention of the onset of wrinkles in a human subject wherein the capsules as defined in any of claims 1 to 7 or the compositions as defined in any of claims 8 to 9 are applied to the skin of said subject.

## Patentansprüche

1. Kapsel mit einem mittleren Durchmesser von weniger als 1 µm, umfassend Alginat, Lecithin, ein zweiwertiges Metallkation und ein Pendlerpeptid.

2. Kapsel nach Anspruch 1, wobei es sich bei dem Peptid um ein Peptid der Formel (I) handelt:
R₁-L-Lys-L-Thr-L-Thr-L-Lys-L-Ser-OH (R₁-SEQ ID NO: 1) (I)
wobei R₁ für eine C₈-C₂₂-Acylgruppierung steht.

3. Kapsel nach Anspruch 2, wobei es sich bei der R₁-Gruppierung um eine Palmitoyl- oder Octanoylgruppierung handelt.

4. Kapsel nach einem der vorhergehenden Ansprüche, wobei es sich bei dem Lecithin um Sojalecithin handelt.

5. Kapsel nach einem der vorhergehenden Ansprüche, **dadurch gekennzeichnet, dass** es sich bei dem zweiwertigen Metallkation um ein Calcium-Kation handelt.

6. Kapsel nach einem der vorhergehenden Ansprüche, wobei der Gewichtsanteil an Peptid, bezogen auf Alginat, 0,0001 bis 0,01 Gramm Peptid pro Gramm Alginat beträgt, der Gewichtsanteil an Peptid, bezogen auf Lecithin, 0,0001 bis 0,01 Gramm Peptid pro Gramm Lecithin beträgt und der Gewichtsanteil an Peptid, bezogen auf zweiwertiges Metallkation, 0,1 bis 1 Gramm Peptid pro Gramm zweiwertiges Metallkation beträgt.

7. Kapsel nach einem der vorhergehenden Ansprüche, **dadurch gekennzeichnet, dass** ihr mittlerer Durchmesser 0,1 bis 0,5 µm beträgt.

8. Zusammensetzung, umfassend die Kapsel gemäß einem der vorhergehenden Ansprüche und Wasser, wobei die Wassermenge vorzugsweise mindestens 90 % (w/w) Wasser, bezogen auf die Summe des Gewichts von Wasser und Kapseln in der Zusammensetzung, beträgt.

9. Zusammensetzung nach Anspruch 8, **dadurch gekennzeichnet, dass** sie ferner ein kosmetisches Mittel aus der Gruppe bestehend aus Tensiden, Feuchtigkeitsspendern, Antioxidantien, Emollientien, Konservierungsstoffen, Feuchthaltemitteln, Viskositätsmodifikatoren und Mischungen davon umfasst, wobei die Zusammensetzung vorzugsweise in Form einer Creme, eines Serums, einer Emulsion, eines Gels, eines Schaums, einer Paste, eine Salbe, einer Milch oder einer Lösung vorliegt.

10. Verwendung einer Kapsel gemäß einem der Ansprüche 1 bis 7 bei der Herstellung einer Zusammensetzung gemäß einem der Ansprüche 8 bis 9.

11. Kosmetische Verwendung der Kapsel gemäß einem der Ansprüche 1 bis 7 oder einer Zusammensetzung gemäß einem der Ansprüche 8 bis 9 für die Hautpflege.

12. Verwendung nach Anspruch 11, wobei es sich bei der Hautpflege um die kosmetische Behandlung von Falten und/oder die kosmetische Prävention des Auftretens von Falten handelt.

13. Verfahren zum Erhalt der Kapsel gemäß einem der Ansprüche 1 bis 7, das Folgendes umfasst:
(a) Herstellen einer wässrigen Suspensionen, die Lecithin und das Peptid umfasst;
(b) Herstellen einer wässrigen Lösung, die eine Alginatquelle umfasst,
(c) Mischen der in Schritt (a) erhaltenen Suspension mit der in Schritt (b) erhaltenen Lösung und
(d) Zugeben einer wässrigen Lösung, die eine Quelle eines zweiwertigen Metallkations umfasst, zu der in Schritt (c) erhaltenen Mischung.

14. Verfahren nach 13, wobei es sich bei der Alginatquelle von Schritt (b) um Natriumalginat handelt und es sich bei der Quelle eines zweiwertigen Metallkations um Calciumchlorid handelt.

15. Verfahren zur kosmetischen Behandlung von Falten und/oder zur kosmetischen Prävention des Auftretens von Falten bei einem menschlichen Individuum, bei dem man die Kapseln gemäß einem der Ansprüche 1 bis 7 oder die Zusammensetzungen gemäß einem der Ansprüche 8 bis 9 auf der Haut des Individuums aufbringt.

## Revendications

1. Capsule possédant un diamètre moyen inférieur à 1 µm comprenant un alginate, une lécithine, un cation métallique divalent et un pentapeptide.

2. Capsule selon la revendication 1, le peptide étant un peptide de formule (I) :
R₁-L-Lys-L-Thr-L-Thr-L-Lys-L-Ser-OH (R₁-SEQ ID NO: 1) (I)
R₁ étant un fragment C₈₋₂₂ acyle.

3. Capsule selon la revendication 2, le fragment R₁ étant un fragment palmitoyle ou octanoyle.

4. Capsule selon l'une quelconque des revendications précédentes, la lécithine étant une lécithine de soja.

5. Capsule selon l'une quelconque des revendications précédentes, **caractérisée en ce que** le cation métallique divalent est un cation calcium.

6. Capsule selon l'une quelconque des revendications précédentes, la proportion en poids de peptide par rapport à l'alginate étant de 0,0001 à 0,01 gramme de peptide par gramme d'alginate, la proportion en poids de peptide par rapport à la lécithine étant de 0,0001 à 0,01 gramme de peptide par gramme de lécithine et la proportion en poids de peptide par rapport au cation métallique divalent étant de 0,1 à 1 gramme de peptide par gramme de cation métallique divalent.

7. Capsule selon l'une quelconque des revendications précédentes, **caractérisée en ce que** son diamètre moyen est de 0,1 à 0,5 µm.

8. Composition comprenant la capsule telle que définie dans l'une quelconque des revendications précédentes et de l'eau, la quantité d'eau étant préférablement d'au moins 90 % (p/p) d'eau par rapport à la somme du poids d'eau et de capsules dans la composition.

9. Composition selon la revendication 8, **caractérisée en ce qu'**elle comprend en outre un agent cosmétique choisi dans le groupe constitué par des tensioactifs, des agents hydratants, des antioxydants, des émollients, des conservateurs, des humectants, des modificateurs de la viscosité, et des mélanges correspondants, ladite composition étant préférablement sous la forme d'une crème, d'un sérum, d'une émulsion, d'un gel, d'une mousse, d'une pâte, d'un onguent, d'un lait, ou d'une solution.

10. Utilisation de la capsule telle que définie dans l'une quelconque des revendications 1 à 7 dans la préparation d'une composition telle que définie dans l'une quelconque des revendications 8 et 9.

11. Utilisation cosmétique de la capsule telle que définie dans l'une quelconque des revendications 1 à 7 ou d'une composition telle que définie dans l'une quelconque des revendications 8 et 9 pour un soin de la peau.

12. Utilisation selon la revendication 11, le soin de la peau étant le traitement cosmétique de rides et/ou la prévention cosmétique de l'apparition de rides.

13. Procédé pour l'obtention de la capsule telle que définie dans l'une quelconque des revendications 1 à 7, lequel procédé comprenant :
(a) la préparation d'une suspension aqueuse comprenant une lécithine et le peptide ;
(b) la préparation d'une solution aqueuse comprenant une source d'alginate,
(c) le mélange de la suspension obtenue dans l'étape (a) avec la solution obtenue dans l'étape (b), et
(d) l'ajout d'une solution aqueuse comprenant une source de cation métallique divalent au mélange obtenu dans l'étape (c).

14. Procédé selon la revendication 13, la source d'alginate de l'étape (b) étant un alginate de sodium et la source de cation métallique divalent étant le chlorure de calcium.

15. Procédé pour le traitement cosmétique de rides et/ou pour la prévention cosmétique de l'apparition de rides chez un sujet humain, les capsules telles que définies dans l'une quelconque des revendications 1 à 7 ou les compositions telles que définies dans l'une quelconque des revendications 8 et 9 étant appliquées à la peau dudit sujet.


### SEQUENCE LISTING

<110> INFINITEC ACTIVOS, S.L.
<120> PEPTIDES ENCAPSULATED IN LECITHIN AND ALGINATE
<130> P16081PC00
<150> ES P201830491
   <151> 2018-05-22
<160> 4
<170> PatentIn version 3.5
<210> 1
   <211> 5
   <212> PRT
   <213> Artificial Sequence
<220>
   <223> SEQ ID NO: 1
<400> 1
   Lys Thr Thr Lys Ser
   1               5
<210> 2
   <211> 6
   <212> PRT
   <213> Artificial Sequence
<220>
   <223> SEQ ID NO: 2
<400> 2
   Lys Ala Gln Lys Arg Phe
   1 5
<210> 3
   <211> 6
   <212> PRT
   <213> Artificial Sequence
<220>
   <223> SEQ ID NO: 3
<400> 3
   Asp Asp Met Asn Lys Lys
   1 5
<210> 4
   <211> 6
   <212> PRT
   <213> Artificial Sequence
<220>
   <223> SEQ ID NO: 4
<400> 4
   Val Gly Val Ala Pro Gly
   1 5